# FASCICULE DE BREVET EUROPEEN

(11) **EP 0 612 711 B1**
(45) Date de publication et mention de la délivrance du brevet: **16.10.1996**
(21) Numéro de dépôt: 94420044.3
(22) Date de dépôt: 10.02.1994
(51) Int. Cl.: C07C 51/12, C07C 55/14

(54) **Procédé d'hydroxycarbonylation de lactones**
Verfahren zur Hydroxykarbonylierung von laktonen
Process for the hydroxycarbonylation of lactones

(30) Priorité: 26.02.1993 FR 9302483
(43) Date de publication de la demande: 31.08.1994
(73) Titulaire: RHONE-POULENC FIBER & RESIN INTERMEDIATES, 92405 Courbevoie (FR)
(72) Inventeur: Denis, Philippe, F-69150 Decines (FR); Patois, Carl, F-69003 Lyon (FR); Perron, Robert, F-69390 Charly (FR)
(74) Mandataire: Vignally, Noel

(56) Documents cités:
- FR-A- 2 015 740

## Description

La présente invention concerne un procédé d'hydroxycarbonylation de lactones, plus précisément des différentes valérolactones et de leurs isomères, par réaction avec le monoxyde de carbone et l'eau, pour former le diacide correspondant.

L'hydroxycarbonylation des acides penténoïques pour préparer l'acide adipique conduit toujours à la formation de quantités plus ou moins importantes de gamma-valérolactone, comme sous-produit de la réaction.

La valorisation de la gamma-valérolactone (ou 4-méthyl-butyrolactone) représente donc un problème important à résoudre, dans le cadre d'un procédé industriel de préparation d'acide adipique par hydroxycarbonylation des acides penténoïques.

Dans le brevet JP-A-54/092913 il est décrit un procédé de préparation d'acides dicarboxyliques par hydroxycarbonylation de lactones, en présence d'un catalyseur au rhodium et d'un promoteur iodé.

Le brevet EP-A-0 395 038, publié quelques années après le précédent, décrit un procédé semblable caractérisé par le choix d'une zone de température et de pression de monoxyde de carbone, ainsi que par un arrêt rapide de la réaction, dès que l'absorption du monoxyde de carbone diminue rapidement, ce qui permet selon le brevet en question d'obtenir de meilleurs rendements en acide dicarboxylique linéaire.

La présente invention se caractérise par l'utilisation d'iridium, un catalyseur différent de celui qui a été décrit antérieurement pour cette réaction.

Plus spécifiquement, elle consiste en un procédé d'hydroxycarbonylation d'au moins une lactone ayant 5 atomes de carbone, par le monoxyde de carbone et l'eau, caractérisé en ce que l'on opère en présence d'une quantité efficace d'un catalyseur à l'iridium et d'un promoteur iodé ou bromé, le rapport molaire promoteur/lr étant compris entre 0,1/1 et 20/1.

Les lactones qui peuvent être utilisées dans le procédé de l'invention, sont plus particulièrement la gamma-valérolactone (ou méthyl-4 butyrolactone), la delta-valérolactone, la méthyl-2 butyrolactone, I'éthyl-3 propiolactone, I'éthyl-2 propiolactone, la diméthyl-2,3 propiolactone.

Parmi ces lactones, la gamma-valérolactone est la plus intéressante en raison de sa formation dans les procédés d'hydroxycarbonylation de l'acide pentène-3 oïque en acide adipique.

On peut le cas échéant mettre en oeuvre des mélanges des lactones indiquées précédemment; les lactones utilisées peuvent contenir une quantité, qui n'est pas critique, d'autres composés comme les acides penténoïques, l'acide valérique, l'acide adipique, l'acide 2-méthyl-glutarique ou l'acide 2-éthyl-succinique.

Pour le catalyseur à l'iridium nécessaire dans le présent procédé, diverses sources d'iridium sont susceptibles d'être mises en oeuvre.

A titre d'exemples de telles sources d'iridium, on peut citer:
- lr métallique; IrO₂ ; Ir₂O₃ ;
- IrCl₃ ; IrCl₃, 3H₂O ;
- IrBr₃ ; IrBr₃, 3H₂O ;
- Irl₃ ;
- lr₂(CO)₄Cl₂ ; lr₂(CO)₄l₂ ;
- lr₂(CO)₈ ; lr₄(CO)₁₂ ;
- lr(CO)[P(C₆H₅)₃]₂l ;
- lr(CO)[P(C₆H₅)₃]₂Cl ;
- lr[P(C₆H₅)₃]₃l ;
- Hlr[P(C₆H₅)₃]₃(CO) ;
- lr(acac)(CO)₂ ;
- [IrCl(cod)]₂ ;
(acac = acétylacétonate ; cod = cyclooctadiène-1,5).

Les catalyseurs à l'iridium qui conviennent plus particulièrement sont: [lrCl(cod)]₂ , Ir₄(CO)₁₂ et lr(acac)(CO)₂.

La quantité de catalyseur à mettre en oeuvre peut varier dans de larges limites.

En général, une quantité, exprimée en moles d'iridium métal par litre de mélange réactionnel, comprise entre 10⁻⁴ et 1 conduit à des résultats satisfaisants. Des quantités inférieures peuvent être utilisées, mais on observe toutefois que la vitesse de réaction est faible. Des quantités supérieures n'ont des inconvénients qu'au plan économique.

De préférence, la concentration en iridium dans le mélange réactionnel est comprise entre 5.10⁻⁴ et 10⁻¹ mole/litre.

Par promoteur iodé ou bromé, on entend dans le cadre du procédé l'iodure d'hydrogène, le bromure d'hydrogène et les composés organo-iodés et organo-bromés capables de générer respectivement l'iodure d'hydrogène et le bromure d'hydrogène dans les conditions réactionnelles; ces composés organo-iodés et organo-bromés sont plus particulièrement les iodures et les bromures d'alkyle ayant de 1 à 10 atomes de carbone,parmi lesquels l'iodure de méthyle et le bromure de méthyle sont préférés.

Le rapport molaire promoteur/lr est de préférence compris entre 1/1 et 10/1.

La réaction est conduite en phase liquide. La température à laquelle on opère est généralement de 100°C à 300°C et de préférence de 150°C à 250°C.

La pression totale à la température de la réaction peut varier dans de larges limites. La pression partielle du monoxyde de carbone, mesurée à 25°C, est généralement de 0,5 bar à 100 bars et de préférence de 1 bar à 80 bars.

Le monoxyde de carbone utilisé peut être le monoxyde de carbone sensiblement pur ou le monoxyde de carbone de qualité technique, tels qu'ils se trouvent dans le commerce.

L'eau est nécessaire à l'hydroxycarbonylation des lactones. Généralement le rapport molaire eau/lactone se situe entre 0,01 et 10. Une quantité plus importante n'est pas souhaitable en raison de la perte d'activité catalytique observée. Le rapport molaire eau/lactone peut être instantanément inférieur à la valeur minimale indiquée précédemment, par exemple si l'on procède à l'injection continue de l'eau, plutôt qu'à son introduction complète avec les autres charges avant la réaction d'hydroxycarbonylation.

De préférence, le rapport molaire eau/lactone est compris entre 0,05 et 2, la remarque précédente relative à la valeur minimale étant également valable.

Un autre paramètre a également de l'importance; il s'agit de la concentration en eau dans le mélange réactionnel. Cette concentration est de préférence maintenue à une valeur de 0,01 mole/litre à 3 moles/litre et pour une meilleure productivité de la réaction à une valeur de 0,01 mole/litre à 2 moles/litre.

Comme indiqué précédemment, cette concentration peut être à un moment donné inférieure aux limites inférieures indiquées, si l'on procède à l'injection continue de l'eau.

La réaction d'hydroxycarbonylation des lactones est conduite soit dans la lactone elle-même comme milieu liquide de la réaction, soit dans un tiers solvant.

Comme tiers solvant, on peut utiliser notamment les acides carboxyliques aliphatiques, saturés ou insaturés, ou aromatiques, comportant au plus 20 atomes de carbone, dans la mesure où ils sont liquides dans les conditions réactionnelles. A titre d'exemples de tels acides carboxyliques, on peut citer l'acide acétique, l'acide propionique, l'acide butanoïque, l'acide valérique, l'acide adipique, les acides penténoïques, l'acide benzoïque et l'acide phénylacétique.

D'autres familles de solvants peuvent également être utilisés, notamment les hydrocarbures saturés aliphatiques ou cycloaliphatiques et leurs dérivés chlorés, dans la mesure où ils sont liquides dans les conditions réactionnelles. A titre d'exemples de tels solvants, on peut citer le benzène, le toluène, le chlorobenzène, le dichlorométhane, l'hexane et le cyclohexane.

Des mélanges de solvants peuvent être utilisés.

Lorsqu'il est présent dans le mélange réactionnel, le solvant représente par exemple de 10 % à 99 % en volume par rapport au volume total dudit mélange réactionnel et de préférence de 30 % à 90 % en volume.

Le procédé d'hydroxycarbonylation selon l'invention permet de valoriser les lactones à 5 atomes de carbone, notamment la gamma-valérolactone qui est formée en quantités plus ou moins importantes lors de l'hydroxycarbonylation des acides penténoïques. Cela permet une augmentation du rendement global d'un procédé de préparation d'acide adipique à partir du butadiène via les acides penténoïques, l'acide adipique étant l'une des matières premières de la préparation des polyamides 6-6.

Le mélange réactionnel final est traité selon les méthodes classiques utilisées en chimie, afin de séparer les différents composés formés et la lactone n'ayant pas réagi, cette dernière pouvant être soumise à une nouvelle hydroxycarbonylation.

Le procédé selon l'invention peut être mis en oeuvre en continu ou en discontinu. Lorsque l'on opère en continu, les rapports relatifs des réactifs, catalyseur, promoteur et solvant pourront être fixés à une valeur optimale par l'homme du métier, alors que généralement dans un procédé mis en oeuvre en discontinu, ces différents rapports évoluent en fonction de la transformation progressive des réactifs.

Les exemples qui suivent illustrent l'invention.

### EXEMPLE 1

Dans une ampoule de verre de 50 cm³, préalablement purgé à l'argon, on introduit successivement:
- gamma-valérolactone (VAL): 20 mmol
- eau: 20 mmol
- acide acétique (AcOH): 10 cm³
- bromure d'hydrogène (en solution aqueuse à 47 % en poids): 2,1 mmol
- lrCl₃: 0,23 mmol

L'ampoule de verre est placée dans un autoclave de 125 cm³, préalablement purgé à l'argon. L'autoclave est fermé, placé dans un four agité et raccordé à l'alimentation de CO sous pression. On met 5 bar de CO à 25°C, puis on chauffe à 220°C. On ajuste la pression en température à 100 bar à l'aide de CO (ce qui correspond à 54 bar de pression partielle de CO mesurée à 25°C) et cette pression est maintenue pendant 11 heures à 220°C.

On refroidit ensuite l'autoclave, on le dégaze et le mélange réactionnel est analysé par chromatographie en phase gazeuse (CPG) et par chromatographie liquide à haute performance (CLHP).

On obtient les résultats suivants:
- taux de transformation (TT) de VAL: 97 %
- rendement en acide adipique (A1) par rapport à VAL chargée (RR): 43 %
- RR en acide 2-méthyl-glutarique (A2) : 15 %
- RR en acide 2-éthyl-succinique (A3) : 3 %
- RR en acide valérique (PA): 19 %

### EXEMPLE 2

On répète l'exemple 1 avec les différences suivantes:
- pression totale à 220°C de 28 bar (ce qui correspond à une pression partielle de CO à 25°C de 11 bar) au lieu de 100 bar ;
- durée de maintien à 220°C de 2 heures au lieu de 11 heures.

On obtient les résultats suivants:
- taux de transformation (TT) de VAL: 68 %
- RR en acide adipique (A1) : 40 %
- RR en acide 2-méthyl-glutarique (A2): 9 %
- RR en acide 2-éthyl-succinique (A3) : 3 %
- RR en acide valérique (PA) : 14 %
- taux de linéarité (rapport acide adipique formé/ensemble des diacides formés): 77 %
- productivité en acide adipique formé: 58 g.l⁻¹h⁻¹

### EXEMPLE 3

On répète l'exemple 2 en remplaçant l'acide acétique par la gamma-valérolactone elle-même : 11,8 g au total. La pression partielle de CO à 25°C (PCO25°) est de 16 bar.

On obtient les résultats suivants :
- taux de transformation (TT) de VAL: 11 %
- rendement en acide adipique (A1) par rapport à VAL transformée (RT) : 26 %
- RT en acide 2-méthyl-glutarique (A2) : 10 %
- RT en acide 2-éthyl-succinique (A3): 14 %
- RT en acide valérique (PA): 15 %

### EXEMPLES 4 A 9

On répète l'exemple 1, avec les différences indiquées dans le tableau ci-après.

Les résultats obtenus sont rassemblés dans le tableau ci-après.

Les abréviations utilisées dans le tableau sont celles qui ont été indiquées précédemment dans les exemples.

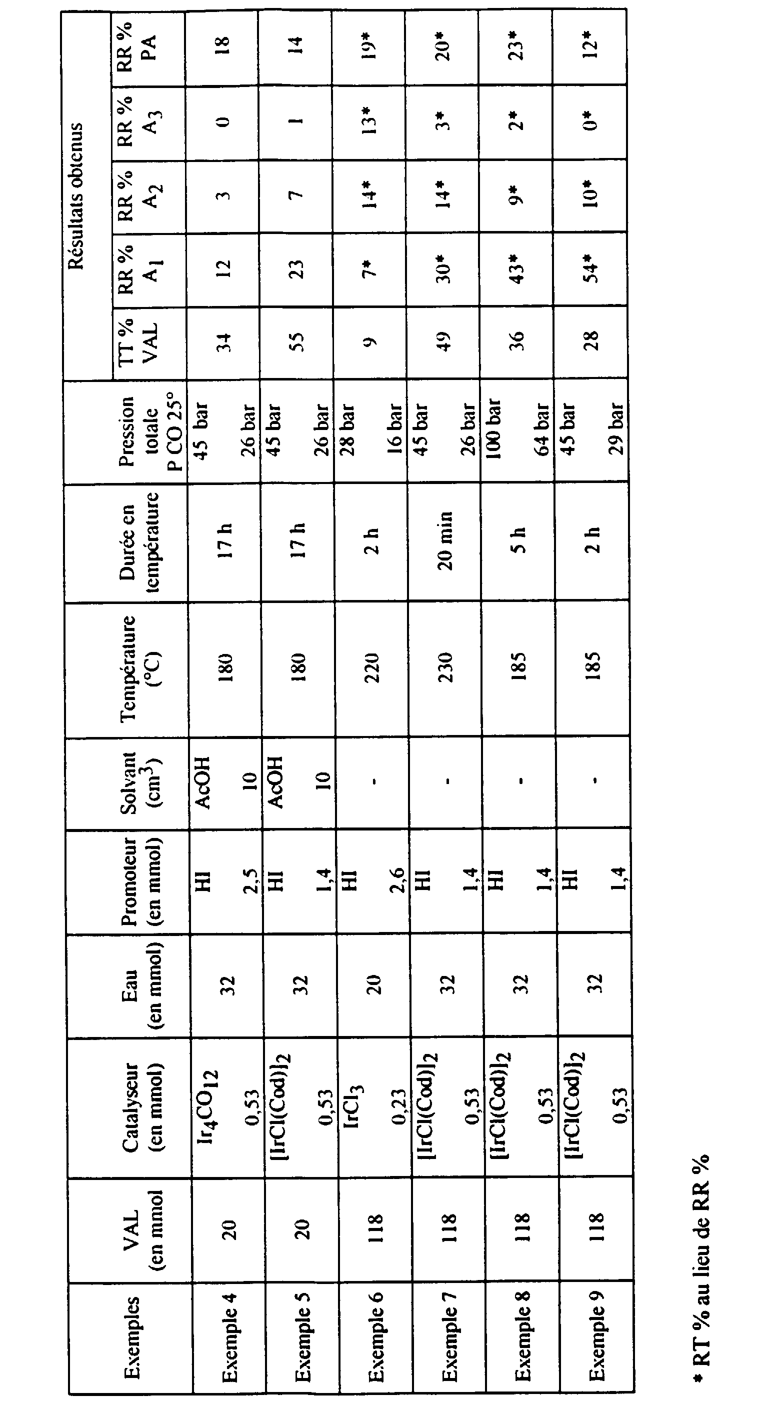

### EXEMPLE 10

On répète l'exemple 1, avec les charges et les conditions opératoires suivantes :
- gamma-valérolactone (VAL): 110 mmol
- eau totale: 12 mmol
- iodure d'hydrogène (en solution aqueuse à 57 % en poids): 0,384 mmol
- lrCl(cod)]₂ : 0,156 mmol

La concentration initiale en eau est de 1 mole/litre.

La température est de 200°C ; la pression totale en température est de 50 bar (soit une pression partielle de CO à 25°C de 30 bar) ; la durée de la réaction en température est de 1 heure.

On obtient les résultats suivants :
- taux de transformation (TT) de VAL: 16 % (soit 100 % de transformation de l'eau)
- rendement en acide adipique (A1) par rapport à VAL transformée (RT) : 52 %
- RT en acide 2-méthyl-glutarique (A2) : 10 %
- RT en acide 2-éthyl-succinique (A3) : 3 %
- RT en acide valérique (PA) : 8 %
- RT en acides penténoïques: 15 %
- RT en acide méthyl-2 butyrique: 1 %
- productivité en A1: 118 9.l⁻¹h⁻¹
- taux de linéarité en A1: 83 %

### EXEMPLE 11

On répète l'exemple 10, avec 24 mmol d'eau au lieu de 12 mmol.

La concentration initiale en eau est de 2 mol/litre.

La durée de la réaction est de 3 heures.

On obtient les résultats suivants :
- taux de transformation (TT) de VAL: 17 %
- RT en A1 : 48 %
- productivité en A1: 40 g.l⁻¹h⁻¹
- taux de linéarité: 74 %

### ESSAI COMPARATIF 1

On répète l'exemple 2 en remplaçant lrCl₃ par la même quantité molaire de RhCl₃; l'essai est maintenu 11 h à 220°C afin d'obtenir un taux de transformation comparable à celui de l'exemple 2.

On obtient les résultats suivants :
- taux de transformation (TT) de VAL: 79 %
- RR en acide adipique (A1) : 51 %
- RR en acide 2-méthyl-glutarique (A2) : 21 %
- RR en acide 2-éthyl-succinique (A3) : 3 %
- RR en acide valérique (PA) 2 %
- productivité en A1: 14 g.h⁻¹l⁻¹
- taux de linéarité: 68 %

On note que le taux de linéarité est plus élevé avec l'iridium (exemple 2) qu'avec le rhodium (77 % contre 68 %).

En outre, l'essai avec le rhodium, malgré une durée de réaction beaucoup plus importante (11 h contre 2 h) conduit à une productivité en acide adipique très nettement plus faible que l'exemple 2 avec l'iridium.

## Revendications

1. Procédé d'hydroxycarbonylation d'au moins une lactone ayant 5 atomes de carbone, par le monoxyde de carbone et l'eau, caractérisé en ce que l'on opère en présence d'une quantité efficace d'un catalyseur à l'iridium et d'un promoteur iodé ou bromé, le rapport molaire promoteur/lr étant compris entre 0,1/1 et 20/1.

2. Procédé selon la revendication 1, caractérisé en ce que la lactone utilisée est choisie parmi la gamma-valérolactone, la delta-valérolactone, la méthyl-2 butyrolactone, l'éthyl-3 propiolactone, l'éthyl-2 propiolactone, la diméthyl-2,3 propiolactone et est de préférence la gamma-valérolactone.

3. Procédé selon l'une des revendications 1 ou 2, caractérisé en ce que le catalyseur à l'iridium mis en oeuvre est choisi parmi:
- Ir métallique; IrO₂ ; Ir₂O₃ ;
- IrCl₃ ; IrCl₃, 3H₂O ;
- IrBr₃ ; IrBr₃; 3H₂O;
- IrI₃ ;
- Ir₂(CO)₄Cl₂ ; Ir₂(CO)₄I₂ ;
- Ir₂(CO)₈ ; Ir₄(CO)₁₂ ;
- Ir(CO)[P(C₆H₅)₃]₂I ;
- Ir(CO)[P(C₆H₅)₃]₂CI ;
- Ir[P(C₆H₅)₃]₃I ;
- HIr[P(C₆H₅)₃]₃(CO) ;
- Ir(acac)(CO)₂ ;
- [lrCI(cod)]₂.

4. Procédé selon l'une des revendications 1 à 3, caractérisé en ce que la quantité de catalyseur, exprimée en moles d'iridium métal par litre de mélange réactionnel, est comprise entre 10⁻⁴ et 1 et de préférence ést comprise entre 5.10⁻⁴ et 10⁻¹ mole/litre.

5. Procédé selon l'une des revendications 1 à 4, caractérisé en ce que le promoteur iodé ou bromé est choisi parmi l'iodure d'hydrogène, le bromure d'hydrogène et les composés organo-iodés et organo-bromés capables de générer respectivement l'iodure d'hydrogène et le bromure d'hydrogène dans les conditions réactionnelles.

6. Procédé selon l'une des revendications 1 à 5, caractérisé en ce que le rapport molaire promoteur/lr est compris entre 1/1 et 10/1.

7. Procédé selon l'une des revendications 1 à 6, caractérisé en ce que la réaction est conduite en phase liquide à une température de 100°C à 300°C et de préférence de 150°C à 250°C et sous une pression partielle du monoxyde de carbone, mesurée à 25°C, de 0,5 bar à 100 bars et de préférence de 1 bar à 80 bars.

8. Procédé selon l'une des revendications 1 à 7, caractérisé en ce que le rapport molaire eau/lactone se situe entre 0,01 et 10 et de préférence est compris entre 0,05 et 2.

9. Procédé selon l'une des revendications 1 à 8, caractérisé en ce que la réaction d'hydroxycarbonylation des lactones est conduite dans la lactone elle-même comme milieu liquide de la réaction.

10. Procédé selon l'une des revendications 1 à 8, caractérisé en ce que la réaction d'hydroxycarbonylation des lactones est conduite dans un tiers solvant choisi parmi les acides carboxyliques aliphatiques, saturés ou insaturés, ou aromatiques, comportant au plus 20 atomes de carbone, les hydrocarbures saturés aliphatiques ou cycloaliphatiques et leurs dérivés chlorés, qui sont liquides dans les conditions réactionnelles.

11. Procédé selon la revendication 10, caractérisé en ce que le solvant représente de 10 % à 99 % en volume par rapport au volume total du mélange réactionnel et de préférence de 30 % à 90 % en volume

12. Procédé selon l'une des revendications 1 à 11, caractérisé en ce que la concentration en eau dans le mélange réactionnel est maintenue à une valeur de 0,01 mole/litre à 3 moles/litre et de préférence de 0,01 mole/litre à 2 moles/litre.

## Claims

1. Process for the hydroxycarbonylation of at least one lactone having 5 carbon atoms by carbon monoxide and water, characterized in that the procedure is carried out in the presence of an effective quantity of an iridium catalyst and of an iodinated or brominated promoter, the promoter/Ir molar ratio being between 0.1:1 and 20:1.

2. Process according to Claim 1, characterized in that the lactone used is chosen from gamma-valerolactone, delta-valerolactone, 2-methylbutyrolactone, 3-ethylpropiolactone, 2-ethylpropiolactone and 2,3-dimethylpropiolactone and is preferably gamma-valerolactone.

3. Process according to one of Claims 1 and 2, characterized in that the iridium catalyst used is chosen from:
- metallic Ir; IrO₂; Ir₂O₃;
- IrCl₃; IrCl₃.3H₂O;
- IrBr₃; IrBr₃.3H₂O;
- IrI₃;
- Ir₂(CO)₄Cl₂; Ir₂(CO)₄I₂;
- Ir₂(CO)₈; Ir₄(CO)₁₂;
- Ir(CO)[P(C₆H₅)₃]₂I;
- Ir(CO)[P(C₆H₅)₃]₂Cl;
- Ir[P(C₆H₅)₃]₃I;
- HIr[P(C₆H₅)₃]₃(CO);
- Ir(acac)(CO)₂;
- [Ircl(cod)]₂.

4. Process according to one of Claims 1 to 3, characterized in that the quantity of catalyst, expressed in moles of iridium metal per litre of reaction mixture, is between 10⁻⁴ and 1 and preferably between 5×10⁻⁴ and 10⁻¹ mol/litre.

5. Process according to one of Claims 1 to 4, characterized in that the iodinated or brominated promoter is chosen from hydrogen iodide, hydrogen bromide and the organoiodine and organobromine compounds capable of generating respectively hydrogen iodide and hydrogen bromide under the reaction conditions.

6. Process according to one of Claims 1 to 5, characterized in that the promoter/Ir molar ratio is between 1:1 and 10:1.

7. Process according to one of Claims 1 to 6, characterized in that the reaction is carried out in the liquid phase at a temperature of 100°C to 300°C and preferably from 150°C to 250°C and at a partial pressure of carbon monoxide, measured at 25°C, of from 0.5 bar to 100 bars and preferably from 1 bar to 80 bars.

8. Process according to one of Claims 1 to 7, characterized in that the water/lactone molar ratio lies between 0.01 and 10 and is preferably between 0.05 and 2.

9. Process according to one of Claims 1 to 8, characterized in that the reaction for the hydroxycarbonylation of the lactones is carried out in the lactone itself as the liquid reaction medium.

10. Process according to one of Claims 1 to 8, characterized in that the reaction for the hydroxycarbonylation of the lactones is carried out in a third solvent chosen from saturated or unsaturated aliphatic or aromatic carboxylic acids containing not more than 20 carbon atoms, saturated aliphatic or cycloaliphatic hydrocarbons and their chlorinated derivatives which are liquid under the reaction conditions.

11. Process according to Claim 10, characterized in that the solvent represents from 10 % to 99 % by volume with respect to the total volume of the reaction mixture, and preferably from 30 % to 90 % by volume.

12. Process according to one of Claims 1 to 11, characterized in that the water concentration in the reaction mixture is maintained at a value of 0.01 mol/litre to 3 mol/litre and preferably from 0.01 mol/litre to 2 mol/litre.

## Patentansprüche

1. Verfahren zur Hydroxycarbonylierung zumindest eines Lactons mit 5 Kohlenstoffatomen durch Kohlenmonoxid und Wasser, dadurch gekennzeichnet, daß man in Anwesenheit einer wirksamen Menge eines Iridiumkatalysators und eines jod- oder bromhaltigen Promotors arbeitet, wobei das Promotor/Ir-Molverhältnis zwischen 0,1/1 und 20/1 liegt.

2. Verfahren gemäß Anspruch 1, dadurch gekennzeichnet, daß das verwendete Lacton unter gamma-Valerolacton, delta-Valerolacton, 2-Methylbutyrolacton, 3-Ethylpropiolacton, 2-Ethylpropiolacton, 2,3-Dimethylpropiolacton ausgewählt wird und vorzugsweise gamma-Valerolacton ist.

3. Verfahren gemäß einem der Ansprüche 1 oder 2, dadurch gekennzeichnet, daß der eingesetzte Iridiumkatalysator ausgewählt wird unter:
- metallisches Ir; IrO₂; Ir₂O₃;
- IrCl₃; IrCl₃, 3H₂O;
- IrBr₃; IrBr₃, 3H₂O;
- IrJ₃;
- Ir₂(CO)₄Cl₂; Ir₂(CO)₄J₂;
- Ir₂(CO)₈; Ir₄(CO)₁₂;
- Ir(CO)[P(C₆H₅)₃]₂J;
- Ir(CO)[P(C₆H₅)₃]₂Cl;
- Ir[P(C₆H₅)₃]₃J;
- HIr[P(C₆H₅)₃]₃(CO);
- Ir(acac)(CO)₂;
- [IrCl(cod)]₂.

4. Verfahren gemäß einem der Ansprüche 1 bis 3, dadurch gekennzeichnet, daß die Katalysatormenge, ausgedrückt in Mol metallisches Iridium je Liter Reaktionsgemisch zwischen 10⁻⁴ und 1 beträgt und vorzugsweise zwischen 5 x 10⁻⁴ und 10⁻¹ Mol/l liegt.

5. Verfahren gemäß einem der Ansprüche 1 bis 4, dadurch gekennzeichnet, daß der Jod- oder Brompromotor unter Jodwasserstoff, Bromwasserstoff und den organischen Jod- und organischen Bromverbindungen, die in der Lage sind, unter den Reaktionsbedingungen Jodwasserstoff bzw. Bromwasserstoff zu erzeugen, ausgewählt wird.

6. Verfahren gemäß einem der Ansprüche 1 bis 5, dadurch gekennzeichnet, daß das Promotor/Ir-Molverhältnis zwischen 1/1 und 10/1 liegt.

7. Verfahren gemäß einem der Ansprüche 1 bis 6, dadurch gekennzeichnet, daß die Reaktion in flüssiger Phase bei einer Temperatur von 100 bis 500°C und vorzugsweise 150 bis 250°C und unter einem partiellen Kohlenmonoxiddruck, gemessen bei 25°C, von 0,5 bar bis 100 bar und vorzugsweise 1 bar bis 80 bar durchgeführt wird.

8. Verfahren gemäß einem der Ansprüche 1 bis 7, dadurch gekennzeichnet, daß das Wasser/Lacton-Molverhältnis sich zwischen 0,01 und 10 und vorzugsweise zwischen 0,05 und 2 erstreckt.

9. Verfahren gemäß einem der Ansprüche 1 bis 8, dadurch gekennzeichnet, daß die Hydroxycarbonylierungsreaktion der Lactone in dem Lacton selbst als flüssigem Reaktionsmilieu durchgeführt wird.

10. Verfahren gemäß einem der Ansprüche 1 bis 8, dadurch gekennzeichnet, daß die Hydroxycarbonylierungsreaktion der Lactone in einem dritten Lösungsmittel, ausgewählt unter den gesättigten oder ungesättigten, aliphatischen oder aromatischen Carbonsäuren mit höchstens 20 Kohlenstoffatomen, den aliphatischen oder cycloaliphatischen, gesättigten Kohlenwasserstoffen und deren chlorierten Derivaten, die unter den Reaktionsbedingungen flüssig sind, durchgeführt wird.

11. Verfahren gemäß Anspruch 10, dadurch gekennzeichnet, daß das Lösungsmittel 10 bis 99 Vol-%, bezogen auf das Gesamtvolumen des Reaktionsgemisches, und vorzugsweise 30 bis 90 Vol-% ausmacht.

12. Verfahren gemäß einem der Ansprüche 1 bis 11, dadurch gekennzeichnet, daß die Wasserkonzentration in dem Reaktionsgemisch bei einem Wert von 0,01 Mol/l bis 3 Mol/l und vorzugsweise 0,01 Mol/l bis 2 Mol/l gehalten wird.
